Europäisches Patentamt

European Patent Office

Office européen des brevets

(11) Veröffentlichungsnummer: **0 162 088**
**B1**

(12) **EUROPÄISCHE PATENTSCHRIFT**

(45) Veröffentlichungstag der Patentschrift:
**24.08.88**

(51) Int. Cl.⁴: **A 47 C 21/04**

(21) Anmeldenummer: **85900045.7**

(22) Anmeldetag: **22.11.84**

(86) Internationale Anmeldenummer:
**PCT/DE 84/00249**

(87) Internationale Veröffentlichungsnummer:
**WO 85/02331 (06.06.85** Gazette **85/13)**

(54) **LIEGE MIT WÄRMESPENDER.**

(30) Priorität: **23.11.83 DE 3342180**

(43) Veröffentlichungstag der Anmeldung:
**27.11.85 Patentblatt 85/48**

(45) Bekanntmachung des Hinweises auf die Patenterteilung:
**24.08.88 Patentblatt 88/34**

(84) Benannte Vertragsstaaten:
**AT BE CH FR GB LI LU NL SE**

(56) Entgegenhaltungen:
**EP - A - 0 071 211**
**CH - A - 471 565**
**DE - C - 263 355**
**DE - C - 513 955**
**US - A - 3 924 284**

(73) Patentinhaber: **BLUM, Manfred, Dierdorfer Strasse 4,
D-5451 Rüscheid (DE)**

(72) Erfinder: **BLUM, Manfred, Dierdorfer Strasse 4,
D-5451 Rüscheid (DE)**

(74) Vertreter: **Hennig, Peter, Wismarer Strasse 2,
D-5400 Koblenz (DE)**

## Beschreibung

Die Erfindung betrifft eine therapeutische Liege mit Wärmespender gemäß dem Oberbegriff des Patentanspruches.

Im Rahmen einer bekannten Sandbadeanlage (DE-C-263 355) ist zur Behandlung des menschlichen Körpers unter Anwendung von erwärmtem Sand eine Badewanne bekannt, in die zuvor erwärmter Sand eingebracht wird und ggfs. mit Hilfe einer in die Badewanne eingebauten Heizschlange auf einer vorbestimmten Temperatur gehalten werden kann. Mit dem Sandbad soll nach der DE-C-263 355 ein starker Reiz auf die Haut des menschlichen Körpers ausgeübt werden, insbesondere sollen im Sandbad mit Hautleiden behaftete Kranke behandelt werden. Da der Kranke bei dieser auf die Linderung von Hautleiden abgestellten Behandlung mehr oder weniger vollständig vom Sand umgeben ist bei direktem Hautkontakt mit dem Sand, ist nach einer Behandlung zum Zweck der Wiederverwendung des Sandes eine aufwendige Reinigung des Sandes erforderlich. Abgesehen von den im Zuge der Wiederaufbereitung des Sandes für erneute Behandlung zwangsläufig sich ergebenden Verlusten an Wärmeenergie sind aber auch an der Badewanne selbst keine Vorkehrungen getroffen, um Verluste von Wärmeenergie weitestgehend einzuschränken.

Überdies bietet die bekannte Sandbadeanlage aber auch nicht die Möglichkeit einer hygienisch einwandfreien Behandlung einer Mehrzahl von am Stützgerüst des Körpers und dazugehörender Muskulatur erkrankten Patienten ohne die Notwendigkeit einer Reinigung des Sandes nach jeder einzelnen Behandlung.

Der Erfindung liegt die Aufgabe zugrunde, eine Einrichtung der eingangs genannten Art zu schaffen, die mit einem Minimum an Aufwand für Erwärmung bzw. Warmhaltung und Reinigung des Sandes die Behandlung des menschlichen Stützgerüstes und der damit verbundenen Muskulatur auf hygienisch einwandfreie Weise für eine größere Anzahl von Patienten ermöglicht, ohne daß dazu der für die Behandlung verwendete Sand erneuert bzw. gereinigt werden müßte. Außer der Wärmezufuhr zum Körper des Patienten, insbesondere also im Bereich des Rückens, soll auch eine Nutzung der Behandlungseinrichtung für eine anschließende, insbesondere Massage-Behandlung oder für Ruhezwecke möglich sein.

Diese Aufgabe wird durch die im kennzeichnenden Teil des Patentanspruches angegebenen Maßnahmen gelöst. Der locker in das Behältnis gefüllte Sand kann sich zusammen mit der lose aufliegenden Abdeckung leicht an die Körperformen anpassen, und zwar unter hygienischen Bedingungen, die eine mehrfache Verwendung der Einrichtung ohne Wechsel oder Reinigung der Sandfüllung ermöglichen. Der Verschlußdeckel für die Einrichtung bietet dabei zum einen die Möglichkeit einer an die Wärmebehandlung anschließenden Behandlung, insbesondere einer Massage-Behandlung oder eine Ruhemöglichkeit. Überdies bildet der Verschlußdeckel mit Polsterung zusammen mit der die Sandfüllung im übrigen umgebenden Wärmeisolation eine die Sandfüllung praktisch rundum abschließende Isolation, wodurch der Energieverbrauch für die Erwärmung und insbesondere Warmhaltung des Sandes auf einem Minimum gehalten werden kann.

Ein Ausführungsbeispiel der Erfindung ist anhand der Zeichnung erläutert.

Es zeigen

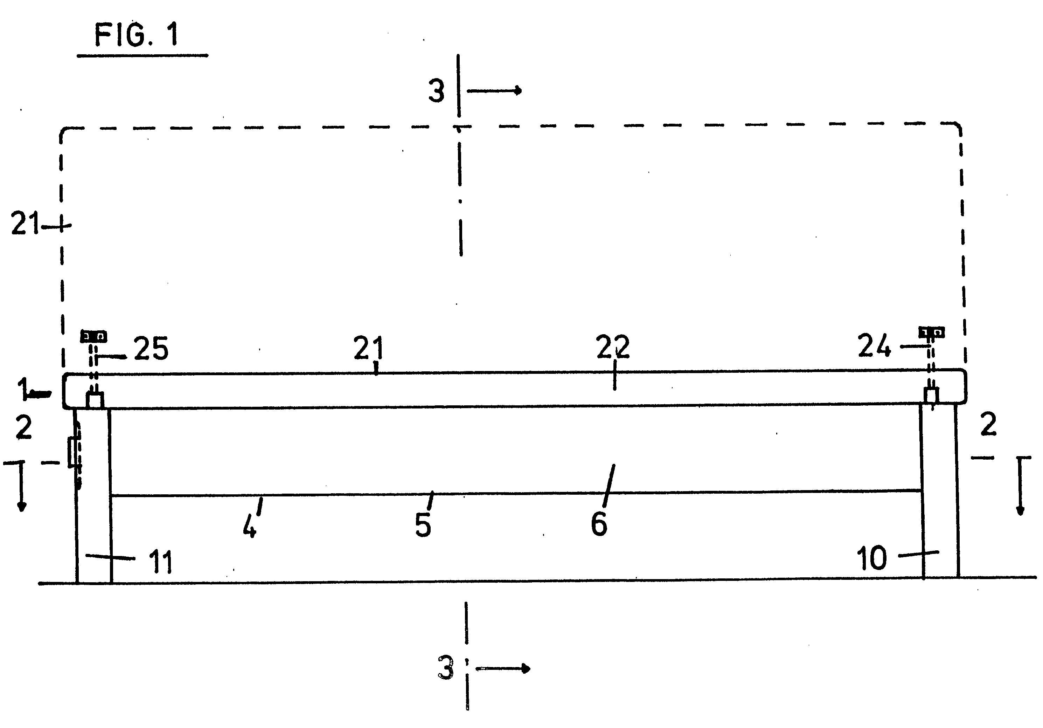

Figur 1 eine erfindungsgemäße therapeutische Behandlungseinrichtung in der Vorderansicht,

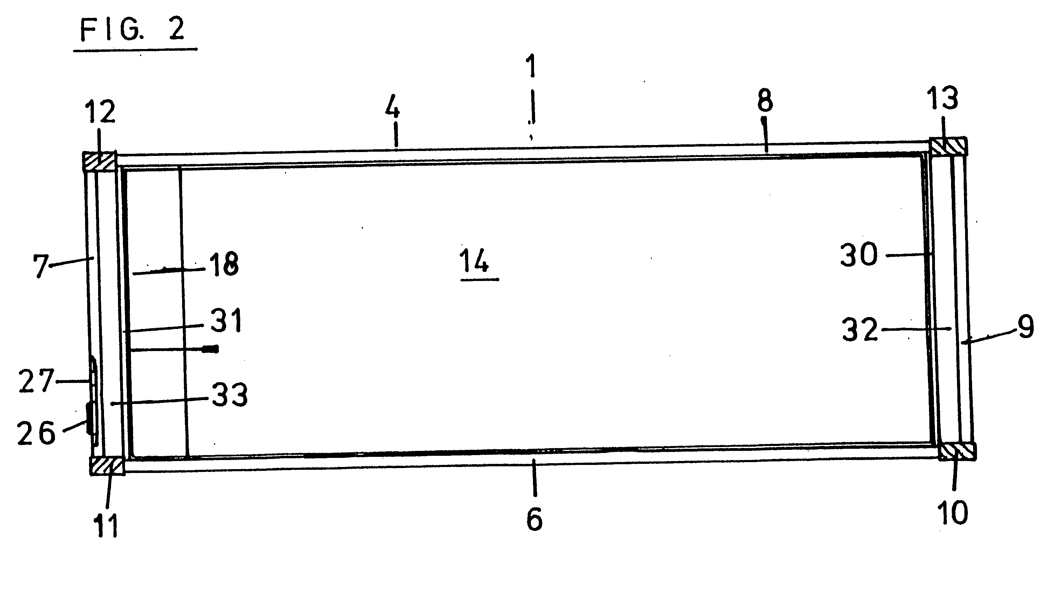

Figur 2 die Behandlungseinrichtung nach Figur 1 in der Draufsicht im Schnitt entsprechend Schnittlinie 2–2 in Figur 1 und

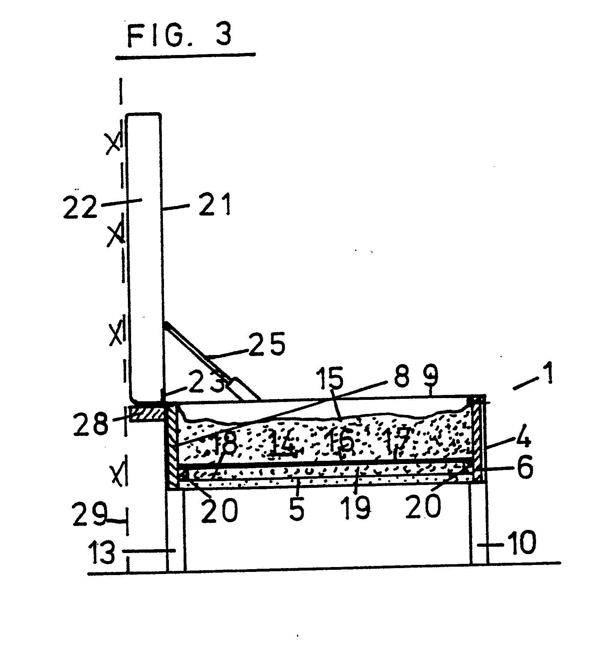

Figur 3 die Behandlungseinrichtung nach Figur 1 in der Seitenansicht im Schnitt entsprechend Schnittlinie 3–3 in Figur 1.

Die Zeichnung zeigt eine Behandlungseinrichtung 1, die im wesentlichen aus einem Behältnis 4 für eine Sandfüllung 14 aus Quarzsand besteht. Das Behältnis 4 weist einen z.B. aus Spanplatte gefertigten Boden 5 auf, der zusammen mit vorzugsweise aus Holz bestehenden Seitenwangen 6, 7, 8 und 9 sowie Eckbeinen 10, 11, 12 und 13 das Behältnis 4 bildet. Die Seitenwangen 6 bis 9 und die Eckbeine 10 bis 13 sind mittels Leim und Langholzdübeln fest miteinander verbunden. Der Boden 5 und zusätzliche, mit 20 bezeichnete Verstärkungsleisten werden mittels Leim und zusätzlich geeigneten Klammern untereinander und mit den Seitenwangen 6 bis 9 fest verbunden.

In der Nähe des Bodens 5 des Behältnisses 4 ist eine vorzugsweise elektrisch betriebene Heizeinrichtung 16 vorgesehen, die nach Art einer an sich bekannten Flächenheizung ausgeführt ist. Derartige Flächenheizungen können z.B. mit elektrisch leitfähigem Material, wie z.B. Kohlenstaub, beschichtete und im übrigen isolierende Folien sein, oder z.B. auch mäanderförmig gelegte und zweckmäßig in einem Traggewebe befestigte Drähte aus elektrisch leitfähigem Material sein. Über der im unteren Bereich der Sandfüllung 14 befindlichen Heizeinrichtung 16 ist bei Auslegung als elektrische Heizeinrichtung ein Erdungsgitter 17 vorgesehen, um die Sandfüllung 14 in jedem Fall und insbesondere im Fall eines Fehlers der Heizeinrichtung elektrisch spannungsfrei zu halten. Die Heizeinrichtung 16 ist mit einem nicht dargestellten Regler versehen, der geeignet ist, die Sandfüllung 14 auf einer vorwählbaren Temperatur zu halten.

Zur Vermeidung unnötiger Wärmeverluste ist unterhalb der Heizeinrichtung 16 und über einer der weiteren Isolation dienenden Styroporfüllung 19 oder einer Füllung aus ähnlich isolierendem Material eine Wärmestrahlung reflektierende, zweckmäßig mit Kunststoff beschichtete Aluminiumfolie 18 im Sandbett 14 vorgesehen. Die Aluminiumfolie 18 ist zweckmäßig an den Seitenwangen 6 bis 9 mindestens bis zur Oberkante der Sandfüllung 14 hochgezogen und dort an den Seitenwangen auf nicht näher beschriebene Weise mittels Leisten oder dgl. befestigt.

Ferner ist an den Seitenwangen 6 bis 9 eine vorzugsweise aus – genügend dichtem – textilem Material bestehende Abdeckung 15 befestigt, die lose auf der Sandfüllung 14 liegt. – Eine unter Zwischenschaltung dieser Abdeckung 15 auf der Sandfüllung 14 liegende Person kommt nicht in direkten Kontakt mit der Sandfüllung; andererseits erlaubt die lose auf dem Sand liegende Abdeckung 15 dennoch eine weitestmögliche Anpassung des die Wärme übertragenden Sandes an die Körperkonturen der betreffenden Person.

Als Verschlußmittel und im Hinblick auf eine zusätzliche Nutzungsmöglichkeit der erfindungsgemäßen Liege – insbesondere nach erfolgter Wärmebehandlung – ist am Behältnis 4 mittels Scharnieren 23 oder ähnlichen Gelenken ein Verschlußdeckel 21 angeordnet. Der Verschlußdeckel 21 weist eine Polsterung 22 auf, so daß sich eine uneingeschränkte Nutzungsmöglichkeit der erfindungsgemäßen Liege auch im Hinblick auf die reine Liegenfunktion ergibt. Darüber hinaus hilft der Verschlußdeckel 21 unnötige Wärmeverluste des erwärmten Sandes weitgehend zu vermeiden.

Um den Verschlußdeckel 21 bei Benutzung der Behandlungseinrichtung in einer passenden Stellung geöffnet zu halten, sind zweckmäßig Stützen 24 und 25 vorgesehen, die einerseits an Teilen, insbesondere Seitenwangen des Behältnisses 4 und andererseits am Verschlußdeckel befestigt sind und die zweckmäßig als an sich bekannte Gasfedern ausgebildet sind.

Zur Aufnahme der einenends mit der Seitenwange 7 bzw. 9 und anderenends mit dem Verschlußdeckel 21 gelenkig verbundenen Stützen 24 und 25 sind innerhalb des Behältnisses 4 mittels je eines der betreffenden Seitenwange 7 bzw. 9 zugeordneten Querholzes 30 bzw. 31 Räume 32 bzw. 33 geschaffen. Die Querhölzer 30 und 31 reichen bis zum Boden des Behältnisses 4 und sind beispielsweise mittels Leim an den Enden mit der betreffenden Seitenwange 6 bzw. 8 und auf der einen Längsseite mit dem Boden 5 verbunden. – Die der Aufnahme der Stützen 24 und 25 dienenden Räume 32 und 33 verhindern somit eine Verschmutzung und eventuelle Funktionsbehinderung der Stützen 24 und 25.

Weiterhin zeigt die Zeichnung noch einen elektrischen Thermostat 26 und einen elektrischen Anschluß 27 in schematischer Darstellung, die hier beispielsweise an der Seitenwange 7 befestigt sind. Der Thermostat 26 reicht mit seinem nicht näher bezeichneten Fühler durch das Querholz 31 hindurch genügend weit in die Sandfüllung 14 hinein. Anstelle der Anbringung des Thermostaten 26 und des elektrischen Anschlusses 27 und ggfs. weiterer elektrischer Mittel auf oder an der Seitenwange 7 ist eine Lösung zweckmäßig, bei der diese elektrischen Einrichtungen und Mittel in einem Gerätekasten zusammengefaßt sind, der dann als Einheit in eine entsprechend große Ausnehmung beispielsweise der Seitenwange 7 eingesetzt wird.

## Patentanspruch

Therapeutische Liege mit Wärmespender zur Behandlung des menschlichen Körpers unter Anwendung von erwärmtem Sand, in Form eines kastenartigen, nach oben zu offenen und im übrigen geschlossenen, mit dem Sand gefüllten Behältnisses, sowie einer innerhalb der Sandfüllung befindlichen Wärmequelle,
gekennzeichnet durch
a) eine lose auf der Sandfüllung (14) liegende Abdeckung (15), die an den Seitenwangen (6 bis 9) des Behältnisses (4) befestigt ist,
b) ein die Sandfüllung (14) bis auf die von der Abdeckung (15) abgedeckte Fläche der Sandfüllung (14) umgebende Wärmeisolation (Aluminiumfolie 18, Styroporfüllung 19) und
c) einen um mindestens etwa 90 Grad aus einer die Sandfüllung (14) bzw. das Behältnis (4) nach oben zu abdeckenden Stellung in eine die Sandfüllung (14) mit Abdeckung (15) freigebende Stellung schwenkbaren Verschlußdeckel (21), der auf der der Sandfüllung gegenüberliegenden Seite mit einer Polsterung (22) versehen ist.

## Claim

Heating bed for the treatment of pain using warmed sand, built in the form of a box-like container, which is only open from above, but otherwise closed, and which is filled with the sand and having a source of heat incorporated within the sand-filling,
characterized by
a) a cover (15) lying slack on the sand-filling (14), and which is fixed to the side-pieces (6–9) of the container (4),
b) a warmth-isolation (aluminium-foil 18, styropor-filling 19) surrounding the sand-filling with the exception of its upper surface, and
c) a locking-cap (21), which can be turned upwardly to at least 90 degrees from the sand-filling (14) or the container (4), and which is to be padded on the opposite side to the sand-filling (14).

## Revendication

Couche chauffante destinée au traitement thérapeutique du corps humain par utilisation de sable chauffé se présentant sous forme d'un coffre s'ouvrant uniquement vers le haut, rempli de sable et comportant une source de chaleur interne,
caractérisé par
a) une couverture amovible (15) reposant sur la masse de sable (14) avec fixation aux parois (6 à 9) du coffre (4),
b) une isolation thermique (feuille d'aluminium 18, polystyrène expansé 19) enveloppant la masse de sable (14) à l'exception de la surface couverte (14),
c) un couvercle (21) se rabattant vers le haut et réglable jusqu'à au moins 90 degrés par rapport à la surface de la masse de sable (14), couvercle pourvu d'un rembourrage (22) sur sa surface extérieure.

FIG. 1

0 162 088

FIG. 2

FIG. 3